# EUROPEAN PATENT SPECIFICATION

(11) **EP 0 441 004 B1**
(45) Date of publication and mention of the grant of the patent: **06.09.1995**
(21) Application number: 90203449.5
(22) Date of filing: 20.12.1990
(51) Int. Cl.: C07C 253/00

(54) **Process for preparing aromatic nitriles**
Verfahren zur Herstellung aromatischer Nitrile
Procédé de préparation de nitriles aromatiques

(30) Priority: 07.02.1990 IT 1928290
(43) Date of publication of application: 14.08.1991
(73) Proprietor: ENICHEM SYNTHESIS S.p.A., 90139 Palermo (IT)
(72) Inventor: Ribaldo, Carlo, I-20131 Milan (IT)
(74) Representative: Fusina, Gerolamo

(56) References cited:
- DE-B- 1 914 688
- CHEMICAL ABSTRACTS vol. 84, no. 25, 21 June 1976, page 539, abstract no. 179843m, Columbus, Ohio, US; Y. TAKIKAWA et al.: "The synthesis of benzonitrile from the reaction of alpha,alpha,alpha- trichlorotoluene with liquid ammonia"

## Description

The present invention relates to a process for preparing aromatic nitriles.

The aromatic nitriles are products known in the art and used in particular as intermediates in the sector of dyes, in the pharmaceutical sector and in the sector of plant medicines.

Several processes are known for producing organic nitrites. Exemplary thereof are:
* reaction of an aromatic halo-derivative with a metal cyanide;
* Sandmayer's reaction on anilines;
* Substitution of an aromatic ring by reaction with Cl-CN (Friedel-Kraft's reaction);
* Ammono-oxidation of aromatic hydrocarbons bearing at least one methyl group;
* Dehydration of benzoic amides; and
* Condensation of benzaldehydes with hydroxylamine and dehydration of the so obtained oxime.

For this techniques known from the prior art, reference is made in particular to the description by K. Friedrich and K. Wallenfels in PATAI - Chemistry of Cyano group, Chapter 2.

The above processes known from the prior art suffer from one or more of the following disadvantages: use of toxic reactants such as cyanides, with very serious waste disposal problems; relatively low reaction yields, in particular as regards the Friedel-Kraft's reaction; high costs of raw materials, in particular in connection with the use of aldehydes and hydroxylamine; and drastic reaction conditions imposed by the process of ammono-oxidation, with consequent metal corrosion problems.
Takikawa et al. (Fac. Eng., Iwate Univ., Morioka Japan, Nippon Kagaku Kaishi, 1976, 4, 637-41, Japan), for example, teaches the synthesis of benzonitrile by the reaction of α,α,α,-trichlorotoluene with liquid ammonia. He also describes the effect, in the reaction, of additives such as water, ammonium chloride, ammonium bromide, sodium chloride, potassium iodide, metallic sodium, copper powder and copper acetate.
However, ammonia is a toxic reactant and it requires drastic reaction condition (high pressure). Furthermore, the use of ammonia brings to the formation of benzamidine hydrochloride and ammonium chloride. Both these by-products affect negatively the benzonitrile yield.

The present Applicant has found now a simple and advantageous process which makes it possible aromatic nitrites to be prepared with high yields and selectivities, by starting from organic trichloro-methyl derivatives and ammonium chloride, the disadvantages of the processes known from the prior art being hence overcome.

In accordance therewith, the present invention relates to a process for preparing aromatic nitriles having the general formula (I): 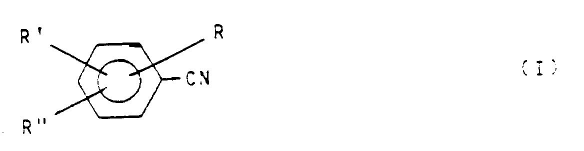
wherein:
- - R: represents the hydrogen atom, a fluorine, chlorine, bromine, or iodine atom, a (C₁-C₅)-alkoxy group, a carboxy group, a (C₁-C₅)-carboxy-alkyl, or an aryl group selected from among phenyl, halo-phenyl, phenyl-ester or nitrile;
- - R' and R'': either equal to, or different from, each other, independently represent the hydrogen atom or a fluorine, chlorine, bromine or iodine atom;
characterized in that an aromatic trichloromethyl derivative having formula (II): 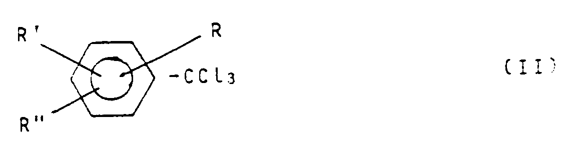
wherein R, R' and R'' have the above set forth meaning, with the exception that when R in formula (I) represents the nitrile group, the corresponding R group in formula (II) represents the trichloromethyl group, is reacted with ammonium chloride, in bulk or in the presence of a non-reactive solvent, with a molar ratio of the ammonium salt to the aromatic trichloro-methyl derivative comprised within the range of from 1:1 to 3:1, at a temperature comprised within the range of from 150°C to 300°C. with a time of the order of from 2 to 6 hours, by operating in the presence of a catalyst consisting of an oxide or a salt of a metal belonging to the groups 8, 11, 12 and 13 (respectively corresponding to groups VIIIA, and I, II, and IIIB) of the Periodic Table of Elements and under condition of removal or blocking of the acid developed as reaction by-product.

Examples of aromatic nitriles (I) which can be prepared by the process according to the present invention are:
- para-chloro-benzonitrile;
- meta-chloro-benzonitrile;
- ortho-chloro-benzonitrile;
- 2-fluoro-benzonitrile;
- 2,3-di-chloro-benzonitrile;
- 3,4-di-chloro-benzonitrile;
- 2,3,4-tri-chloro-benzonitrile;
- 2,4,5-tri-chloro-benzonitrile;
- 3,4,5-tri-chloro-benzonitrile;
- 2,5,6-tri-chloro-benzonitrile;
- 3,4,6-tri-chloro-benzonitrile;
- terephthalonitrile; and
- isophthalonitrile.

In the reaction, the process is carried out with a molar ratio of the ammonium chloride to the aromatic trichloro-methyl derivative (II) comprised within the range of from 1:1 to 3:1, and preferably of from 1:1 to 2:1, at a temperature comprised within the range of from 150 to 300°C and preferably of from 180 to 230°C, operating in the presence of a catalyst consisting of an oxide or a salt of a metal belonging to the groups VIIIA and I, II, IIIB, (IUPAC classification) of the Periodic Table of Elements. In particular, the sulfate, halide, acetate and carbonate salts may be used. For the intended purpose, copper and zinc halides and in particular chlorides are preferred.

The catalyst is advantageously used in amounts comprised within the range of from 0.01 to 5% by weight, and preferably of the order of 0.5% by weight, relatively to the weight of the aromatic trichloromethyl derivative.

The reaction is furthermore carried out under conditions of removal or blocking of the acid which is developed as reaction byproduct. For example, if ammonium chloride is used, hydrogen chloride acid is developed according to the reaction schematically reported hereunder:

Ar-CCl₃ + NH₄Cl → Ar-CN + 4 HCl

in which Ar represents the aromatic radical of the nitrile.

The reaction can be carried out in bulk or in the presence of a suitable non-reactive solvent generally selected from the group consisting of the aromatic hydrocarbons, aromatic chlorocarbons and nitriles.

By operating under the above specified conditions, the necessary times for a complete or nearly complete reaction of compound (II) are comprised within the range of from about 2 hours to about 6 hours, and typically are of the order of 4 hours.

By operating according to the process of the present invention, practically complete conversions of compound (II) are accomplished, with values of selectivity to the aromatic nitrite (III) equal to, or higher than 97% by mol.

By the process according to the present invention, mono-nitriles or di-nitriles can be obtained, as a function of the meaning of R in formula (II).

The aromatic nitrite obtained can be easily separated from the reaction mixture, and, in particular, from the catalyst and the excess of the ammonium salt, by known techniques, such as distillation and solvent treatment. In particular, the aromatic nitrile can be directly distilled off from the reaction mixture, or it can be extracted by dissolution in a suitable organic solvent. In any case, the catalyst and the ammonium salt can be easily recovered and recycled to a subsequent reaction cycle, a feature which makes it possible the catalyst to be used again and the ammonium salt used to be exhausted.

The following experimental examples are supplied to better illustrate the present invention.

### Example 1

The following reactants:

| | |
|---|---|
| - ortho-chloro-benzotrichloride | 167 g (0.7264 mol) |
| - ammonium chloride | 40 g (0.7476 mol) |
| - copper-(II) chloride | 8.3 g (0.061 mol) |

are charged to a reactor of 300 ml, equipped with temperature-control jacket, stirring means, reflux condenser and bottom drain.

The reaction mixture is stirred and heated up to 220-225°C. Under these conditions, ortho-chloro-benzonitrile is formed, and hydrogen chloride is developed as reaction byproduct. The reaction mixture is kept stirred at said temperature until the development of hydrogen chloride ceases. The reaction time is of about 4 hours.

The inner reactor temperature is then decreased down to 80°C, the reaction mixture is discharged in 500 g of toluene and, the resulting mixture is filtered when cold to separate the solids constituted by copper-(II) chloride and the excess of ammonium chloride. Toluene is evaporated off from the filtrate and a residue consisting of 97.8 g (0.7112 mol) of ortho-chloro-benzonitrile is obtained. The conversion of ortho-chloro-benzotrichloride is hence of 100%, and the selectivity to ortho-chloro-benzonitrile is of 98% (melting paint 46°C).

The so obtained ortho-chloro-benzonitrile can be used as obtained, or it can be submitted to a preliminary distillation.

### Example 2

The process is carried out with the same apparatus described in Example 1, to which a feeding dip tube is added. The following reactants are added to the reactor:

| | |
|---|---|
| - Ortho-chloro-benzotrichloride | 167 g (0.7264 mol) |
| - Ammonium chloride | 40 g (0.7476 mol) |
| - Copper-(II) chloride | 8.3 g (0.061 mol) |

The reaction is carried out as described in Example 1 and at the end or the reaction, 40 g (0.7476 mol) of ammonium chloride is added. The temperature of the mass is increased up to 210°C, and the addition of ortho-chloro-benzo-trichloride through the dip tube is started. Said addition is carried out by means of a metering pump and the addition rate is of about 40 g/hour (26 ml/hour; 0.1739 mol/hour).

Under these conditions, a nearly immediate conversion of ortho-chloro-benzotrichloride into ortho-chloro-benzonitrile with development of hydrogen chloride gas takes place.

The temperature of the reaction mass is stabilized at about 205°C, until the end of the addition of ortho-chloro-benzotrichloride (addition time = about 4 hours).

The reflux condenser is then excluded and through a valve a jacketed ampoul is connected with the reactor through a horizontal condenser. Vacuum is applied to the whole system, and ortho-chloro-benzonitrile is evaporated by operating at 520 mm_{Hg} and 185°C and is collected inside the ampoul. A conversion of 100%, referred to ortho- chloro-benzotrichloride is thus accomplished. The selectivity to ortho-chloro-benzonitrile is of 98%.

### Example 3

The process is carried as described in Example 1. The following reactants are added to the reactor:

| | |
|---|---|
| - Para-chloro-benzotrichloride | 167 g (0.7264 mol) |
| - Ammonium chloride | 78 g (1.4579 mol) |
| - Copper-(II) chloride | 8.3 g (0.061 mol) |

The reaction mixture is stirred and heated up to 230°C. Under these conditions, para-chloro-benzonitrile is formed and hydrogen chloride develops as reaction by-product. The reaction mixture is kept stirred at the specified temperature until the development of hydrogen chloride ceases. The reaction time is of about 5 hours.

The internal temperature of the reactor is then decreased down to 100°C, the reaction mixture is discharged in 500 g of toluene and is filtered when cold to separate ammonium chloride, rameic chloride and a solid byproduct which can be assigned to a trimer.

The cake on the filter is washed with water to dissolve the present inorganic compounds. Toluene is evaporated off from the filtrate and as the residue para-chloro-benzonitrile is obtained. The conversion of para-chloro-benzotrichloride is of 100%, with a selectivity to para-chloro-benzonitrile (melting point 90-91°C) of 97%.

### Example 4

The process is carried as described in Example 3. The following reactants are added to the reactor:

| | |
|---|---|
| - Para-chloro-benzotrichloride | 167 g (0.7264 mol) |
| - Ammonium chloride | 78 g (1.4579 mol) |
| - Zinc chloride | 8.3 g (0.0609 mol) |

By operating under the same conditions as of Example 3, the conversion of para-chloro-benzotrichloride is of 100%, with a selectivity to para-chloro-benzonitrile of 99%. The yield in the trimer by-product is about 1%.

### Example 5

The process is carried as described in Example 1. The following reactants are added to the reactor:

| | |
|---|---|
| - 2,4-dichloro-benzotrichloride | 264.5 g (1.0 mol) |
| - Ammonium chloride | 53.5 g (1.0 mol) |
| - Copper-(II) chloride | 13.2 g (0.098 mol) |

The reaction mixture is stirred and heated up to 230°C. Under these conditions, 2,4-dichloro-benzonitrile is formed and hydrogen chloride develops as reaction by-product. The reaction mixture is kept stirred at the above specified temperature until the development of hydrogen chloride ceases. The reaction time is of about 2.5 hours.

The internal temperature of the reactor is then decreased down to 80°C, the reaction mixture is discharged in 500 g of toluene and filtered when cold to separate the excess of rameic chloride and ammonium chloride.

Toluene is evaporated off from the filtrate and as the residue 2,4-dichloro-benzonitrile is obtained. The conversion of 2,4-dichloro-benzotrichloride is hence of 100% and the selectivity to 2,4-dichloro-benzonitrile (melting point 61-62°C) is of 99%.

## Claims

1. Process for preparing aromatic nitriles having the general formula (I): 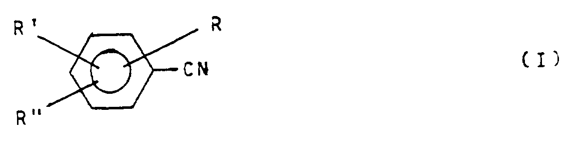 wherein:
- R represents the hydrogen atom, a fluorine, chlorine, bromine or iodine atom, a (C₁-C₅)-alkoxy group, a carboxy group, a (C₁-C₅)-carboxy-alkyl, or an aryl group selected from among phenyl, halo-phenyl, phenyl-ether or nitrile;
- R' and R'' either equal to, or different from, each other, independently represent the hydrogen atom or a fluorine, chlorine, bromine, or iodine atom;
characterized in that an aromatic trichloromethyl derivative having formula (II): 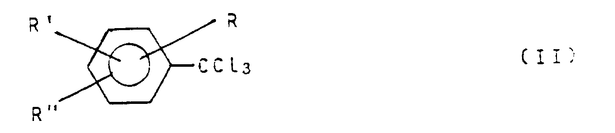 wherein R, R' and R'' have the above set forth meaning, with the exception that when R in formula (I) represents the nitrile group, the corresponding R group in formula (II) represents the trichloromethyl group, is reacted with ammonium chloride, in bulk or in the presence of a non-reactive solvent, with a molar ratio of the ammonium salt to the aromatic trichloro-methyl derivative comprised within the range of from 1:1 to 3:1, at a temperature comprised within the range of from 150°C to 300°C, with a time of the order of from 2 to 6 hours, by operating in the presence of a catalyst consisting of an oxide or a salt of a metal belonging to the groups 8, 11, 12 and 13 (respectively corresponding to groups VIIIA, and I, II, and IIIB) of the Periodic Table of Elements and under condition of removal or blocking of the acid developed as reaction by-product.

2. Process according to claim 1, characterized in that the catalyst is selected from the group consisting of the oxides and sulfate, halide, acetate and carbonate salts of copper and zinc, and is used in amounts comprised within the range of from 0.01 to 5% by weight relatively to the weight of the aromatic trichloromethyl derivative.

3. Process according to claim 2, characterized in that the catalyst is copper or zinc chloride and is used in amounts of approximately 0.5% by weight, relatively to the weight of the aromatic trichloromethyl-derivative.

4. Process according to claim 1, characterized in that the process is carried out with a molar ratio of the ammonium chloride to the aromatic trichloro-methyl derivative comprised within the range of from 1:1 to 2:1, at a temperature comprised within the range of from 180 to 230°C and with a reaction time of the order of 4 hours.

5. Process according to claim 1, characterized in that the following nitriles (I) are prepared:
- para-chloro-benzonitrile;
- meta-chloro-benzonitrile;
- ortho-chloro-benzonitrile;
- 2-fluoro-benzonitrile;
- 2,3-di-chloro-benzonitrile;
- 3,4-di-chloro-benzonitrile;
- 2,3,4-tri-chloro-benzonitrile;
- 2,4,5-tri-chloro-benzonitrile;
- 3,4,5-tri-chloro-benzonitrile;
- 2,5,6-tri-chloro-benzonitrile;
- 3,4,6-tri-chloro-benzonitrile;
- terephthalonitrile; and
- isophthalonitrile.

## Patentansprüche

1. Verfahren zur Herstellung von aromatischen Nitrilen mit der allgemeinen Formel (I): 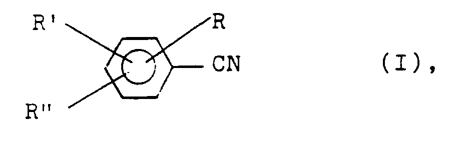 worin:
- R ein Wasserstoffatom, ein Fluor-, Chlor-, Brom- oder Iod-atom, eine (C₁-C₅)-Alkoxygruppe, eine Carboxygruppe, eine (C₁-C₅)-Carboxyalkylgruppe oder eine unter Phenyl, Halogenphenyl, Phenylether ausgewählte Arylgruppe oder Nitril darstellt;
- R' und R'', die gleich oder voneinander verschieden sind, unabhängig voneinander ein Wasserstoffatom oder ein Fluor-, Chlor-, Brom- oder Iodatom bedeuten;
dadurch gekennzeichnet, daß ein aromatisches Trichlormethylderivat mit der Formel (II): 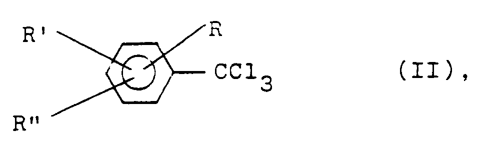 worin R, R' und R'' die oben angegebene Bedeutung besitzen, mit der Ausnahme, daß dann, wenn R in Formel (I) die Nitrilgruppe darstellt, die entsprechende Gruppe R in Formel (II) die Trichlormethylgruppe bedeutet, mit Ammoniumchlorid in der Masse oder in Anwesenheit eines nicht-reaktiven Lösungsmittels mit einem Molverhältnis des Ammoniumsalzes zum aromatischen Trichlormethylderivat im Bereich von 1:1 bis 3:1 bei einer Temperatur im Bereich von 150°C bis 300°C während einer Reaktionsdauer von 2 bis 6 Stunden umgesetzt wird, wobei in Anwesenheit eines Katalysators gearbeitet wird, der aus einem Oxid oder einem Metall aus den Gruppen 8, 11, 12 und 13 (bzw. entsprechend den Gruppen VIIIA und I, II und IIIB) des Periodensystems der Elemente besteht, und unter der Bedingung gearbeitet wird, daß die als Reaktionsnebenprodukt gebildete Säure abgetrennt oder blockiert wird.

2. Verfahren nach Anspruch 1, dadurch gekennzeichnet, daß der Katalysator aus der aus den Oxiden und Sulfat-, Halogenid-, Acetat- und Carbonatsalzen von Kupfer und Zink bestehenden Gruppe ausgewählt und in Mengen im Bereich von 0,01 bis 5 Gew.-%, bezogen auf das Gewicht des aromatischen Trichlormethylderivats, verwendet wird.

3. Verfahren nach Anspruch 2, dadurch gekennzeichnet, daß der Katalysator Kupfer- oder Zinkchlorid ist und in Mengen von ungefähr 0,5 Gew.-%, bezogen auf das Gewicht des aromatischen Trichlormethylderivats, verwendet wird.

4. Verfahren nach Anspruch 1, dadurch gekennzeichnet, daß das Verfahren mit einem Molverhältnis von Ammoniumchlorid zu dem aromatischen Trichlormethylderivat im Bereich von 1:1 bis 2:1 bei einer Temperatur im Bereich von 180 bis 230°C und mit einer Reaktionszeit in der Größenordnung von 4 Stunden ausgeführt wird.

5. Verfahren nach Anspruch 1, dadurch gekennzeichnet, daß die nachstehenden Nitrile (I) hergestellt werden:
- para-Chlor-benzonitril;
- meta-Chlor-benzonitril;
- ortho-Chlor-benzonitril;
- 2-Fluor-benzonitril;
- 2,3-Dichlor-benzonitril;
- 3,4-Dichlor-benzonitril;
- 2,3,4-Trichlor-benzonitril;
- 2,4,5-Trichlor-benzonitril;
- 3,4,5-Trichlor-benzonitril;
- 2,5,6-Trichlor-benzonitril;
- 3,4,6-Trichlor-benzonitril;
- Terephthalonitril; und
- Isophthalonitril.

## Revendications

1. Procédé pour la préparation de nitriles aromatiques répondant à la formule générale (I) : 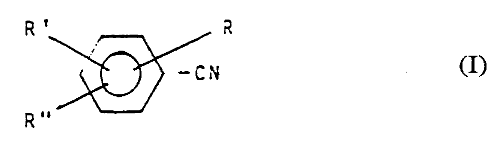 dans laquelle :
- R représente l'atome d'hydrogène, de fluore, de chlore, de brome ou d'iode, un groupe alkoxy en C₁₋₅, un groupe carboxy, un groupe carboxy-alkyle en C₁₋₅, ou un groupe aryle choisi dans l'ensemble formé par les résidus phényle, halogénophényle, phényl-éther ou nitrile;
- R' et R'' à la fois équivalents à, ou différents de, chacun d'entre eux, représentant indépendamment l'atome d'hydrogène ou de fluore, de chlore, de brome ou d'iode;
caractérisé en ce que l'on fait réagir
un dérivé aromatique trichlorométhylé répondant à la formule (II) : 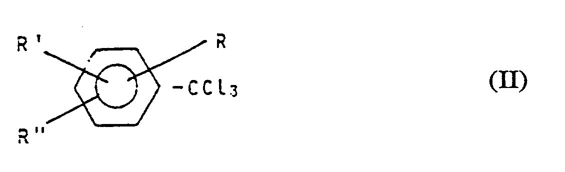 dans laquelle R, R' et R'' ont la même signification que ci-dessus, à l'exeption du fait que, quand dans la formule (I) R représente un groupe nitrile, le groupe R correspondant dans la formule (II) représente un groupe trichlorométhyle,
avec du chlorure d'ammonium, en masse ou en présence d'un solvant inerte, avec un rapport molaire du sel d'ammonium au dérivé aromatique trichlorométhylé compris entre 1:1 et 3:1, à une température comprise entre 150°C et 300°C, pendant un temps de l'ordre de 2 à 6 heures, en opérant en présence d'un catalyseur qui consiste en un oxyde ou un métal appartenant aux groupes 8, 11, 12 et 13 (correspondant respectivement aux groupes VIIIA, et I, II, et IIIB) de la classification périodique des éléments et dans des conditions d'élimination ou de piégeage de l'acide, sous-produit formé au cours de la réaction.

2. Procédé conforme à la revendication 1, caractérisé en ce que le catalyseur est choisi dans le groupe formé par les oxydes et sels de type sulfate, halogénure, acétate et carbonate de cuivre et de zinc, et est utilisé en des quantités comprises entre 0,01% et 5% en poids, rapporté au poids du dérivé aromatique trichlorométhylé.

3. Procédé conforme à la revendication 2, caractérisé en ce que le catalyseur est un chlorure de cuivre ou de zinc et est utilisé en une quantité d'environ 0,5% en poids, rapportée au poids de dérivé aromatique trichlorométhylé.

4. Procédé conforme à la revendication 1, caractérisé en ce que' le procédé est effectué avec un rapport molaire du chlorure d'ammonium au dérivé aromatique trichlorométhylé compris entre 1:1 et 2:1, à une température comprise entre 180°C et 230°C et avec un temps de réaction de l'ordre de 4 heures.

5. Procédé conforme à la revendication 1, caractérisé en ce que les nitriles (I) suivants sont préparés :
- para-chloro-benzonitrile;
- méta-chloro-benzonitrile;
- ortho-chloro-benzonitrile;
- 2-fluoro-benzonitrile;
- 2,3-di-chloro-benzonitrile;
- 3,4-di-chloro-benzonitrile;
- 2,3,4-tri-chloro-benzonitrile;
- 2,4,5-tri-chloro-benzonitrile;
- 3,4,5-tri-chloro-benzonitrile;
- 2,5,6-tri-chloro-benzonitrile;
- 3,4,6-tri-chloro-benzonitrile;
- téréphtalonitrile et
- isophtalonitrile.
